# EUROPEAN PATENT APPLICATION

(11) **EP 2 476 395 A1**
(43) Date of publication of application: **18.07.2012**
(21) Application number: 10815190.3
(22) Date of filing: 07.06.2010
(51) Int. Cl.: A61F 2/28, A61L 27/00

(54) **BONE PROSTHETIC MATERIAL**

(30) Priority: 10.09.2009 JP 2009209124
(71) Applicant: Olympus Terumo Biomaterials Corp., Shinjuku-ku, Tokyo 163-0914 (JP)
(72) Inventor: OISHI, Makoto, Tokyo 163-0914 (JP)
(74) Representative: Schicker, Silvia
(86) International application number: PCT/JP2010/059613
(87) International publication number: WO 2011/030592

(57) **Abstract**

Disclosed is a bone prosthetic material that is capable of preventing a plurality of members from being disconnected due to a difference in the coefficient of thermal expansion, and that is also capable of facilitating the work to insert them while ensuring the bone replacement capability. Provided is a bone prosthetic material (1) comprising: a plurality of prosthetic material pieces (11) and (12) which include bioabsorbable materials having different absorption rates, and which are adjacent and connected to each other; and a joint (14) which is provided in a connecting surface (13) between these prosthetic material pieces (11) and (12), and which is mutually combined to connect the prosthetic material pieces (11) and (12) while limiting displacement in directions along the connecting surface (13).

## Description

### {Technical Field}

The present invention relates to a bone prosthetic material to be embedded into a body of an organism.

### {Background Art}

So far, a method of producing a bone prosthetic material, in which slurries having different concentrations of ceramic particulates are laid in a laminated pattern and integrally sintered, has been known (for example, refer to Patent Literature 1).
Moreover, a vertebra fixing member comprising an osteoconductive material (for example, β-tricalcium phosphate) in which a porous body and a dense body are combined, has been known (for example, refer to Patent Literature 2).
In addition, a bone prosthetic material to be filled in a position of the cortical bone and a position of the cancellous bone, has also been known (for example, refer to Patent Literature 3).

### {Citation List}

### {Patent Literature}

{PTL 1}
   Japanese Unexamined Patent Application, Publication No.2003-180816
{PTL 2}
   Japanese Unexamined Patent Application, Publication No. Hei 10-33656
{PTL 3}
   Japanese Unexamined Patent Application, Publication No.2005-152503

### {Summary of Invention}

### {Technical Problem}

However, the bone prosthetic material disclosed in Patent Literature 1 has a disadvantage in that, during the production by means of integral sintering of a plurality of layers having different concentrations of ceramic particulates, physical stresses may be accumulated in the interface between respective layers due to the difference in the coefficient of thermal expansion, which may culminate in disconnection of these layers or such a problem.

Moreover, because the vertebra fixing member disclosed in Patent Literature 2 is a fixing member to be placed in a space between vertebrae (between bones), the member itself has to have enough strength so as not to be compressed by pressures from bones. Therefore, it is necessary to ensure a certain level of strength by making a part or some parts of the fixing member from a dense body. This gives a disadvantage in that the speed of bone replacement (absorption) is so slow particularly in the dense body that the fixing member can not play enough function as a bone prosthetic material to be placed inside a bone.

In addition, the bone prosthetic material disclosed in Patent Literature 3 has a disadvantage in that: it is necessary to carry out at least two insertion works respectively for the cortical bone and the cancellous bone in the affected part; and, furthermore, it is difficult to accurately insert the bone prosthetic material into the more deeply located cancellous bone. Moreover, because the bone prosthetic material to be inserted into the cancellous bone side is a mixture of a granulated ceramic and a bone marrow, there is also a disadvantage in that it is difficult to insert an accurate amount of the material in the cancellous bone side.

The present invention takes the above-mentioned situations into consideration with an object of providing a bone prosthetic material that is capable of preventing a plurality of members from being disconnected due to the difference in the coefficient of thermal expansion, and that is also capable of facilitating the work to insert them while ensuring the bone replacement capability.

### {Solution to Problem}

The present invention provides the following solutions in order to achieve the above-mentioned object.
The present invention provides a bone prosthetic material comprising: a plurality of prosthetic material pieces which include bioabsorbable materials having different absorption rates, and which are adjacent and connected to each other; and a joint which is provided in a connecting surface between these prosthetic,material pieces, and which is mutually combined to connect the prosthetic material pieces while limiting displacement in directions along the connecting surface.

According to the present invention, it is possible, by filling the bone prosthetic material in a defect created by, for example, osteotomy, so that a prosthetic material piece having a relatively low absorption rate (hereunder, referred to as a "low absorbable piece") is located on the cortical bone side and a prosthetic material piece having a relatively high absorption rate (hereunder, referred to as a "high absorbable piece") is located on the cancellous bone side, to fix the high absorbable piece to the inside of the cancellous bone right after the operation with the aid of the low absorbable piece fixed to the cortical bone. Since the high absorbable piece fixed to the inside of the cancellous bone has a high bioabsorption rate, cells related to bone metabolism can act for angiogenesis, absorption of the material, bone formation, and such an event, by which the high absorbable piece will be replaced with the autologous bone. Thereafter, the low absorbable piece fixed to the inside of the cortical bone will also be replaced with the autologous bone in the same manner. By so doing, the defect created by osteotomy can be quickly repaired.

In this case, because the plurality of the prosthetic material pieces are connected by the joint that limits displacement in directions along the connecting surface, it is possible, by inserting them into the defect in a direction orthogonal to the connecting surface as the insertion direction, to prevent positional misalignment of the plurality of the prosthetic material pieces in directions along the connecting surface.
Moreover, because the plurality of the prosthetic material pieces are connected by the joint, it is possible to respectively form the final shape of each of the plurality of the prosthetic material pieces prior to the connection. This makes it possible to prevent these prosthetic material pieces from being disconnected due to the difference in the coefficient of thermal expansion after the connection.
Further, because the plurality of the prosthetic material pieces are integrated, the work to insert these prosthetic material pieces can be done at once. By so doing, the operation can be facilitated and also the time required therefor can be shortened, by which the burden on the patient can be alleviated.

In the above-mentioned invention, the joint may also comprise: a recess which is provided in one of the adjacent prosthetic material pieces; and a projection which is provided on another one of the adjacent prosthetic material pieces to be engaged with the recess.
By having such a joint, it is possible to connect the adjacent prosthetic material pieces so that displacement in directions along the connecting surface can be prevented in a simple manner. Moreover, it is also possible, by increasing the cross sectional area of the recess towards the bottom face as well as increasing the cross sectional area of the projection towards the top end, to prevent changes of their relative positions not only in directions along the connecting surface but also in directions orthogonal to the connecting surface. Therefore, the workability for insertion into the defect can be improved.

In the above-mentioned invention, a female screw may be formed on the recess and a male screw may be formed on the projection.
By so doing, it is possible to make the adjacent prosthetic material pieces detachable/attachable in a simple manner, and it is also possible to prevent displacement not only in directions along the connecting surface but also in directions orthogonal to the connecting surface. Therefore, the workability for insertion into the defect can be improved.

In the above-mentioned invention, the joint may also comprise: recesses provided in each of the adjacent prosthetic material pieces; and a joining member to be engaged with these recesses to thereby connect these each of the adjacent prosthetic material pieces.
By having such a joint, the material of the joining member can be selected irrespective of the materials of the prosthetic material pieces so that the joining member can have a desired strength to thereby enhance the strength to bond the adjacent prosthetic material pieces.

In the above-mentioned invention, the bone prosthetic material may also be formed in a wedge shape so that the cross sectional area gradually decreases from one end of a prosthetic material piece having a relatively low absorption rate towards one end of a prosthetic material piece having a relatively high absorption rate, among the adjacent prosthetic material pieces.
By so doing, it is possible to improve the insertability into the defect created by, for example, osteotomy, and also it is possible to fix the low absorbable piece having a relatively low absorption rate to the cortical bone in a simple manner.

In the above-mentioned invention, the prosthetic material pieces may also be formed from a calcium phosphate based compound, a calcium sulfate based compound, a calcium carbonate based compound, or a compound in which a part of an element thereof is substituted with another element, or a ceramic which includes a composite of these compounds as a main component.
By so doing, it is possible to effectively replace the prosthetic material pieces with the autologous bone. Therefore, the defect created by osteotomy or the like can be quickly repaired.

The above-mentioned invention may also be such that: a low absorbable piece serving as a prosthetic material piece having a relatively low absorption rate, out of the adjacent prosthetic material pieces, is a porous body whose porosity is 70% or lower; a high absorbable piece serving as a prosthetic material piece having a relatively high absorption rate, out of the adjacent prosthetic material pieces, is a porous body whose porosity is 50% or higher but 90% or lower; and the porosity of the high absorbable piece is higher than the porosity of the low absorbable piece, while the difference in the porosity between them is 10% or larger.
By forming the low absorbable piece and the high absorbable piece from porous bodies having such porosities, the low absorbable piece and the high absorbable piece can be respectively and satisfactorily replaced with the autologous bone. Therefore, the defect created by osteotomy or the like can be quickly repaired. In addition, it is possible, by filling the bone prosthetic material so that the low absorbable piece having a low porosity, in other words, a high level of hardness is located on the cortical bone side, and the high absorbable piece having a high porosity is located on the cancellous bone side, to enhance the strength to fix the low absorbable piece to the cortical bone.

The above-mentioned invention may also be such that: the low absorbable piece includes pores having a radius of 1 nm or larger but smaller than 1000 nm, which account for 80% or more in the volume ratio, and pores having a radius of 1 µm or larger but 100 µm or smaller, which account for less than 20% in the volume ratio; and the high absorbable piece includes pores having a radius of 1 nm or larger but smaller than 1000 nm, which account for 50% or more in the volume ratio, and pores having a radius of 1 µm or larger but 100 µm or smaller, which account for less than 50% in the volume ratio.
The bioabsorption rate decreases as the porosity is lower. Thus, it is effective to increase micropores so as to broaden the surface area. Accordingly, it is possible, by forming the low absorbable piece and the high absorbable piece in the above-mentioned manner, to effectively replace these pieces with the autologous bone. Therefore, the defect created by osteotomy or the like can be quickly repaired.

The above-mentioned invention may also be such that: the compressive strength of a low absorbable piece serving as a prosthetic material piece having a relatively low absorption rate, out of the adjacent prosthetic material pieces, is 10 MPa or higher; the compressive strength of a high absorbable piece serving as a prosthetic material piece having a relatively high absorption rate, out of the adjacent prosthetic material pieces, is 0.1 MPa or higher but 20 MPa or lower; and the compressive strength of the low absorbable piece is higher than that of the high absorbable piece, while the difference in the compressive strength between the low absorbable piece and the high absorbable piece is 5 MPa or higher.
A strength of about 10 MPa is necessary for a low absorbable piece and a high absorbable piece to be pushed in to fill a defect created by osteotomy or the like. In addition, a strength of 0.1 MPa is necessary for them to be formed in a block shape. Accordingly, it is possible, by forming the low absorbable piece and the high absorbable piece in the above-mentioned manner, for the low absorbable piece to be pushed in to fill the defect, as well as being possible for the high absorbable piece to be formed in a block shape.

The above-mentioned invention may also be such that: the bioabsorption rate of a low absorbable piece serving as a prosthetic material piece having a relatively low absorption rate, out of the adjacent prosthetic material pieces, is from 1 to 5 years, provided that the size is 1 cm³; and the bioabsorption rate of a high absorbable piece serving as a prosthetic material piece having a relatively high absorption rate, out of the adjacent prosthetic material pieces, is from 0.25 to 2 years, provided that the size is 1 cm³.
By so doing, the low absorbable piece having a porosity of, for example, 60% can be absorbed in 27 to 37 months, and the high absorbable piece having a porosity of, for example, 75% can be absorbed in 2 years. Therefore, the defect created by osteotomy or the like can be quickly repaired.

The above-mentioned invention may also be such that: the longitudinal length from one end to the other end throughout the plurality of the prosthetic material pieces, after connected, is from 3 mm to 40 mm; the length in a direction orthogonal to the longitudinal direction of the plurality of the prosthetic material pieces, after connected, is from 3 mm to 30 mm; and these pieces are to be filled in a defect created in the cortical bone and the cancellous bone.
By having the plurality of the prosthetic material pieces after connected in such a dimension, it is possible to improve the workability for insertion into the defect created by, for example, osteotomy in the cortical bone and the cancellous bone.

### {Advantageous Effects of Invention}

The present invention offers an effect of enabling to prevent a plurality of members from being disconnected due to a difference in the coefficient of thermal expansion and also enabling to facilitate the work to insert them while ensuring the bone replacement capability.

### {Brief Description of Drawings}

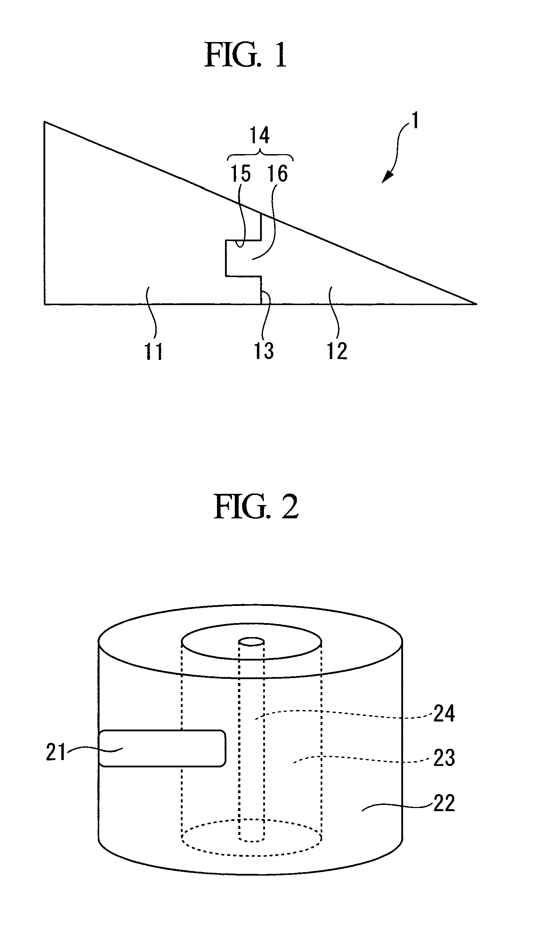

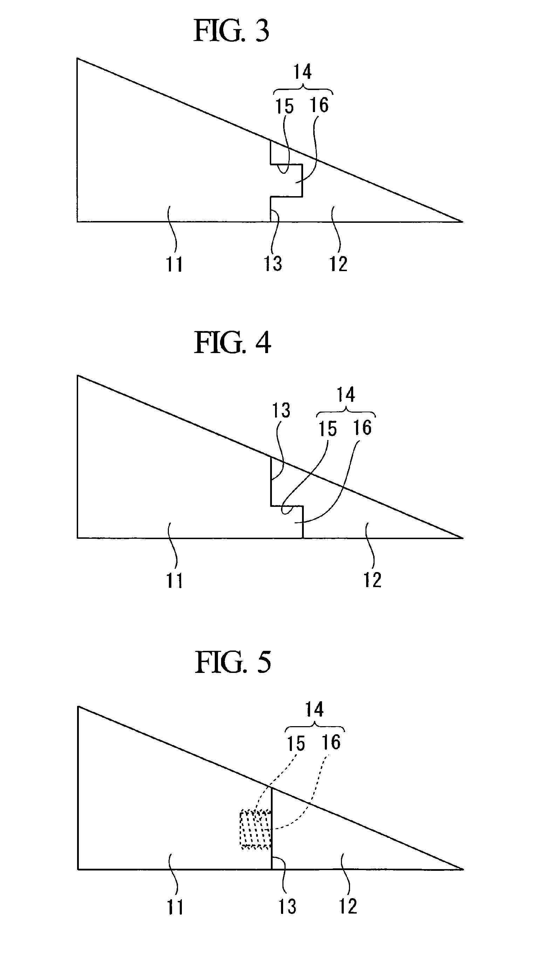

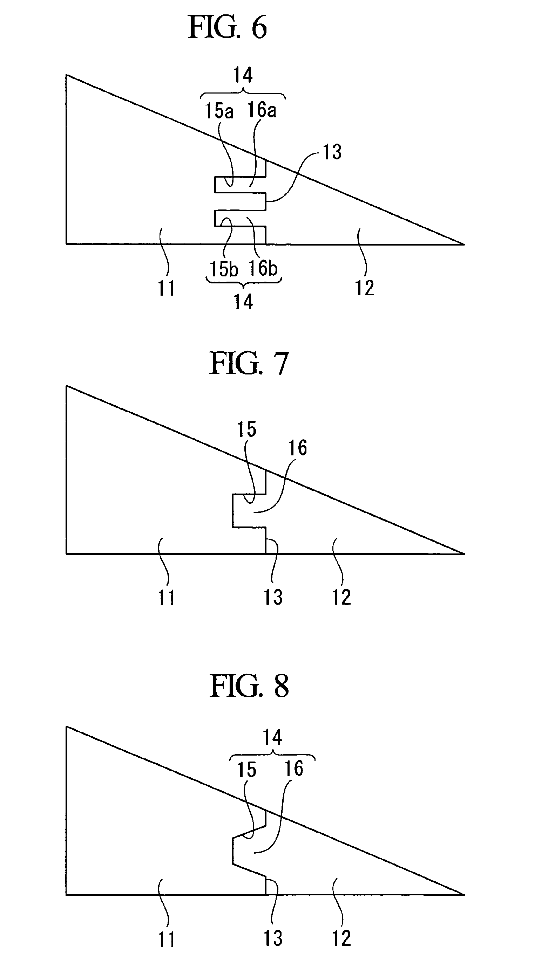

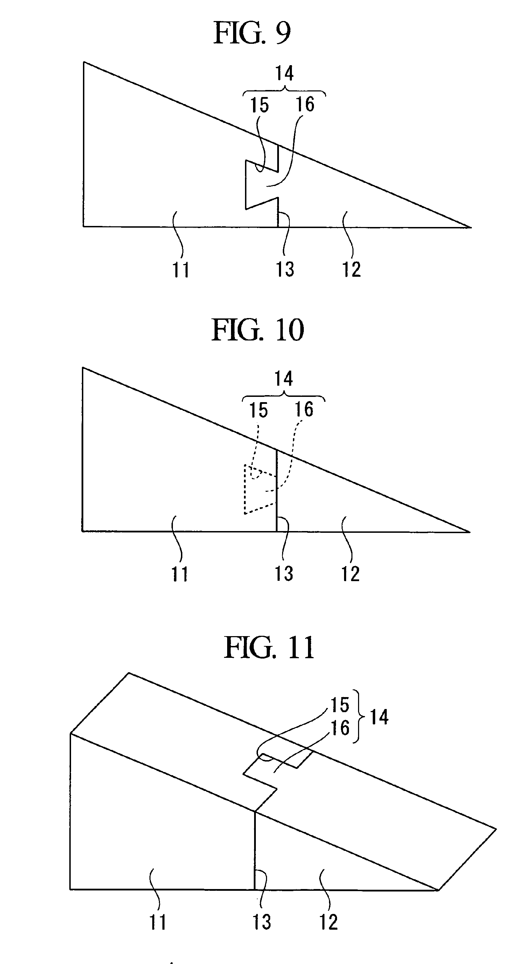

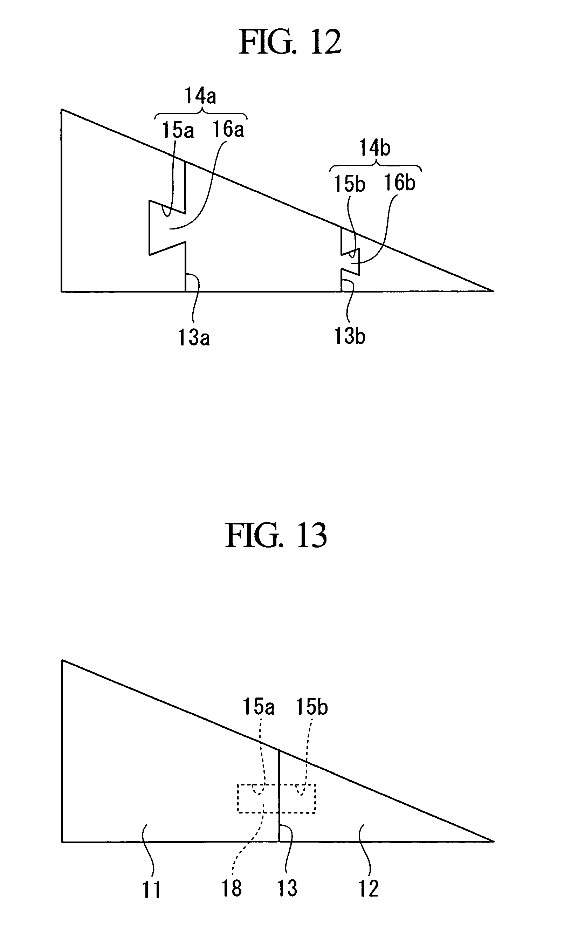

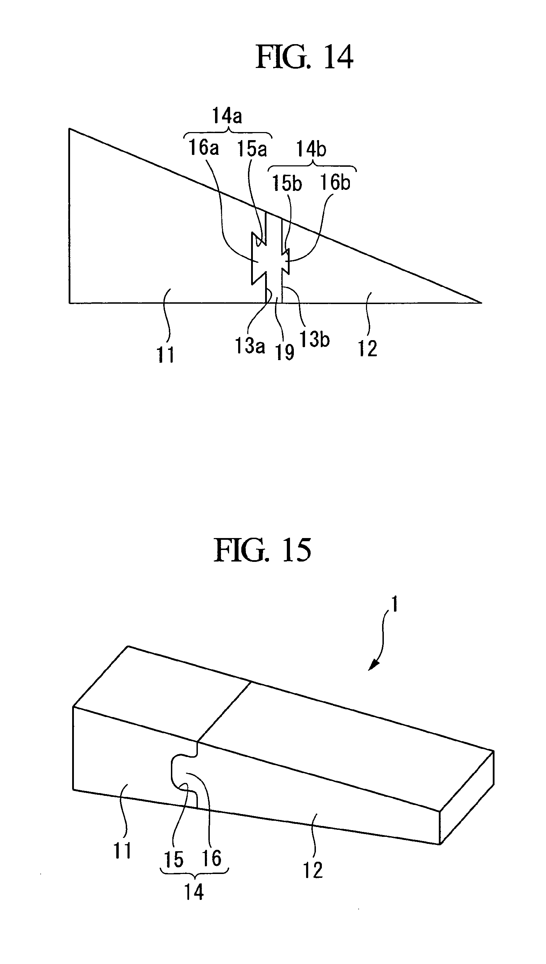

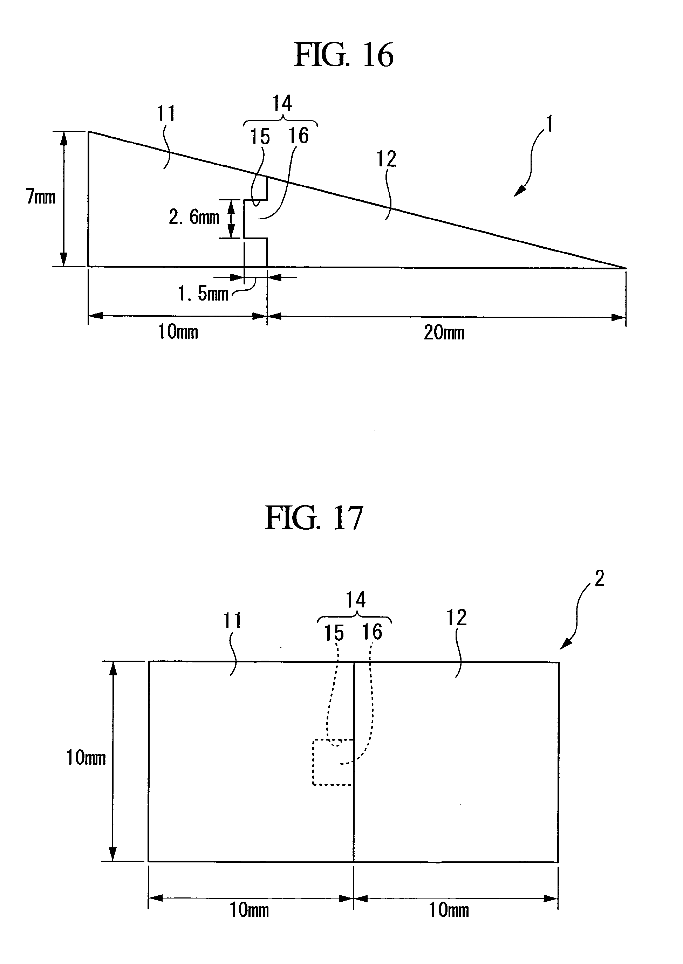

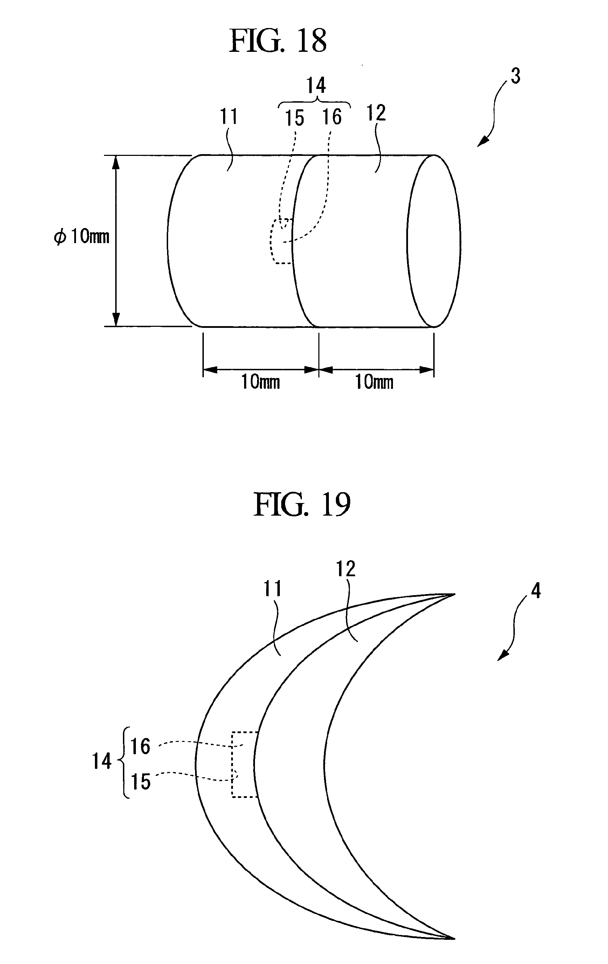

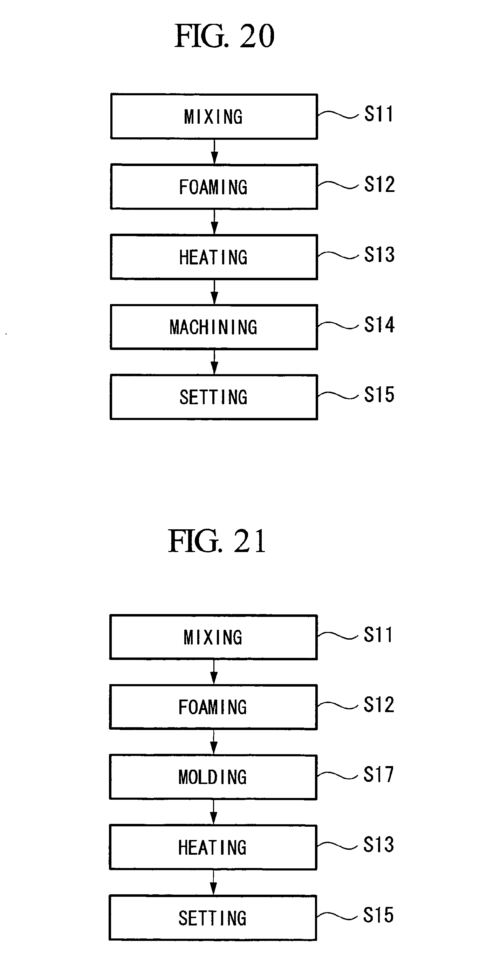

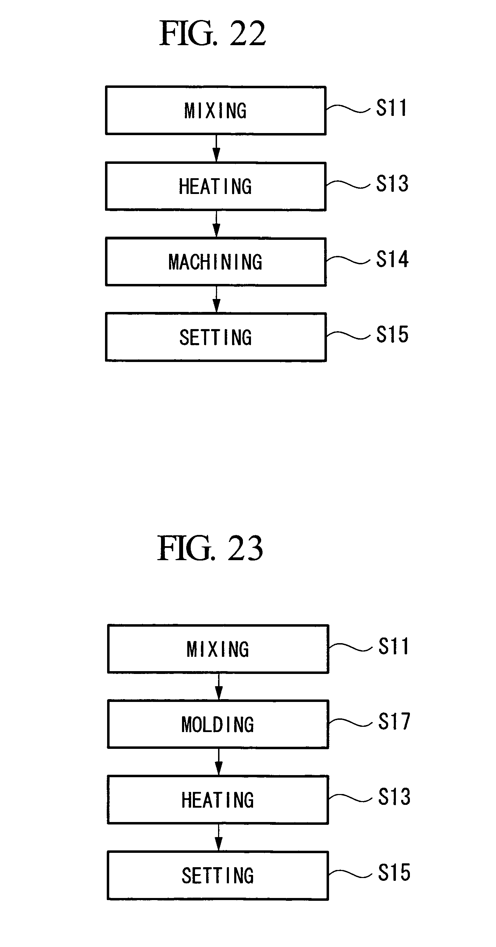

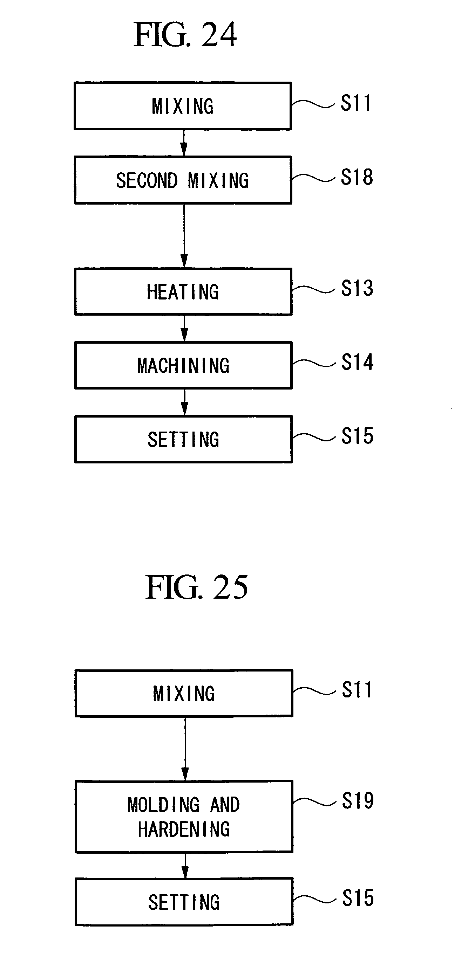

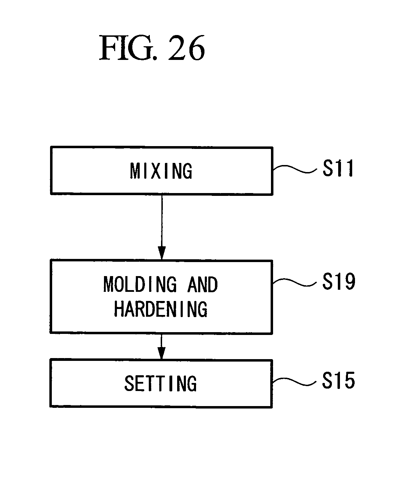

{Fig. 1} Fig. 1 is a transverse sectional view of a bone prosthetic material according to an embodiment of the present invention.
{Fig. 2} Fig. 2 is a schematic view illustrating the internal structure of a human bone.
{Fig. 3} Fig. 3 is a transverse sectional view showing a modified example of the bone prosthetic material of Fig. 1.
{Fig. 4} Fig. 4 is a transverse sectional view showing a modified example of the bone prosthetic material of Fig. 1.
{Fig. 5} Fig. 5 is a transverse sectional view showing a modified example of the bone prosthetic material of Fig. 1.
{Fig. 6} Fig. 6 is a transverse sectional view showing a modified example of the bone prosthetic material of Fig. 1.
{Fig. 7} Fig. 7 is a transverse sectional view showing a modified example of the bone prosthetic material of Fig. 1.
{Fig. 8} Fig. 8 is a transverse sectional view showing a modified example of the bone prosthetic material of Fig. 1.
{Fig. 9} Fig. 9 is a transverse sectional view showing a modified example of the bone prosthetic material of Fig. 1.
{Fig. 10} Fig. 10 is a transverse sectional view showing a modified example of the bone prosthetic material of Fig. 1.
{Fig. 11} Fig. 11 is a transverse sectional view showing a modified example of the bone prosthetic material of Fig. 1.
{Fig. 12} Fig. 12 is a transverse sectional view showing a modified example of the bone prosthetic material of Fig. 1.
{Fig. 13} Fig. 13 is a transverse sectional view showing a modified example of the bone prosthetic material of Fig. 1.
{Fig. 14} Fig. 14 is a transverse sectional view showing a modified example of the bone prosthetic material of Fig. 1.
{Fig. 15} Fig. 15 is a perspective view of a bone prosthetic material according to the first example.
{Fig. 16} Fig. 16 is a transverse sectional view of the bone prosthetic material of Fig. 15.
{Fig. 17} Fig. 17 is a transverse sectional view of a bone prosthetic material according to the second example.
{Fig. 18} Fig. 18 is a side view of a bone prosthetic material according to the third example.
{Fig. 19} Fig. 19 is a top view of a bone prosthetic material according to the fourth example.
{Fig. 20} Fig. 20 is a flowchart showing a method for producing the bone prosthetic material of Fig. 1.
{Fig. 21} Fig. 21 is a flowchart showing a method for producing the bone prosthetic material of Fig. 1.
{Fig. 22} Fig. 22 is a flowchart showing a method for producing the bone prosthetic material of Fig. 1.
{Fig. 23} Fig. 23 is a flowchart showing a method for producing the bone prosthetic material of Fig. 1.
{Fig. 24} Fig. 24 is a flowchart showing a method for producing the bone prosthetic material of Fig. 1.
{Fig. 25} Fig. 25 is a flowchart showing a method for producing the bone prosthetic material of Fig. 1.
{Fig. 26} Fig. 26 is a flowchart showing a method for producing the bone prosthetic material of Fig. 1.

### {Description of Embodiments}

Hereunder is a description of a bone prosthetic material 1 according to one embodiment of the present invention with reference to the drawings.
As shown in Fig. 1 and Fig. 2, the bone prosthetic material 1 according to this embodiment is a member to be inserted to fill a defect 21 created by, for example, osteotomy or the like. The bone prosthetic material 1 comprises bioabsorbable materials.

As shown in Fig. 1, the bone prosthetic material 1 comprises a plurality of prosthetic material pieces 11 and 12 which are composed of bioabsorbable materials having different absorption rates, and which are adjacent and connected to each other. Here, the description will be made by setting a situation in which the bone prosthetic material 1 comprises a low absorbable piece 11 serving as a prosthetic material piece having a relatively low absorption rate and a high absorbable piece 12 serving as a prosthetic material piece having a relatively high absorption rate.

The bone prosthetic material 1 is formed in a wedge shape so that the cross sectional area gradually decreases from one end of the low absorbable piece 11 towards one end of the high absorbable piece 12.
By having such a shape, as shown in Fig. 2, it is possible to improve the insertability into the defect 21 created by, for example, osteotomy, and also it is possible to fix the low absorbable piece 11 to the cortical bone 22 having a high level of hardness in a simple manner and thereby to fix the high absorbable piece 12 to the cancellous bone 23 having a low level of hardness in a simple manner.

The bone prosthetic material 1 has a dimension such that the longitudinal length (depth) from one end to the other end, in other words, the total length of the low absorbable piece 11 and the high absorbable piece 12 is from 3 mm to 40 mm, and the length in a direction orthogonal to the longitudinal direction of Fig. 1, in other words, the height of the low absorbable piece 11 is from 3 mm to 30 mm.
By having the bone prosthetic material 1 in such a dimension, it is possible to improve the workability for insertion into the defect 21 created by, for example, osteotomy in the cortical bone and the cancellous bone. Although the size of the bone varies between individuals, it is particularly preferable that the bone prosthetic material 1 has a height of 7.5 mm to 15 mm and a depth of 25 mm to 35 mm.

As shown in Fig. 1, the bone prosthetic material 1 comprises a joint 14 which is provided in the connecting surface 13 between the low absorbable piece 11 and the high absorbable piece 12, and which is mutually combined to connect the low absorbable piece 11 and the high absorbable piece 12 while limiting displacement in directions along the connecting surface 13.

The joint 14 comprises a recess 15 provided in the low absorbable piece 11 and a projection 16 provided on the high absorbable piece 12 to be engaged with the recess 15. The recess 15 and the projection 16 are extendedly present in the width direction of the low absorbable piece 11 and the high absorbable piece 12 (the direction orthogonal to the plane of Fig. 1).
By providing the joint 14 having such a structure, it is possible to connect the low absorbable piece 11 and the high absorbable piece 12 so that displacement in directions along the connecting surface 13 can be prevented in a simple manner. By so doing, it is possible to improve the workability for insertion of the bone prosthetic material 1 into the defect 21 shown in Fig. 2.

Both the low absorbable piece 11 and the high absorbable piece 12 are formed from a calcium phosphate based compound. By forming them from such a material, it is possible to effectively replace the prosthetic material pieces with the autologous bone. Therefore, the defect 21 created by osteotomy or the like can be quickly repaired.
Note that the low absorbable piece 11 and the high absorbable piece 12 may also be formed from a calcium sulfate based compound, a calcium carbonate based compound, or a compound in which a part of an element thereof is substituted with another element, or a biocompatible substance such as a ceramic which includes a composite of these compounds as a main component.

The low absorbable piece 11 is a porous body whose porosity is 70% or lower, and the high absorbable piece 12 is a porous body whose porosity is 50% or higher but 90% or lower. In addition, the porosity of the high absorbable piece 12 is higher than the porosity of the low absorbable piece 11, while the difference in the porosity between them is set to be 10% or larger.

By forming the low absorbable piece 11 and the high absorbable piece 12 from porous bodies having such porosities, the low absorbable piece 11 and the high absorbable piece 12 can be respectively and satisfactorily replaced with the autologous bone. Therefore, the defect 21 created by osteotomy or the like can be quickly repaired. In addition, it is possible, by filling the bone prosthetic material 1 so that the low absorbable piece 11 having a low porosity, in other words, a high level of hardness, is located on the cortical bone 22 side, and the high absorbable piece 12 having a high porosity is located on the cancellous bone 23 side, to enhance the strength to fix the low absorbable piece 11 to the cortical bone 22.

The low absorbable piece 11 includes pores having a radius of 1 nm or larger but smaller than 1000 nm, which account for 80% or more in the volume ratio, and pores having a radius of 1 µm or larger but 100 µm or smaller, which account for less than 20% in the volume ratio. In addition, the high absorbable piece 12 includes pores having a radius of 1 nm or larger but smaller than 1000 nm, which account for 50% or more in the volume ratio, and pores having a radius of 1 µm or larger but 100 µm or smaller, which account for less than 50% in the volume ratio.

The bioabsorption rate decreases as the porosity is lower. Thus, it is effective to increase micropores so as to broaden the surface area. Accordingly, it is possible, by forming the low absorbable piece 11 and the high absorbable piece 12 in the above-mentioned manner, to effectively replace these pieces with the autologous bone. Therefore, the defect 21 created by osteotomy or the like can be quickly repaired.

The compressive strength of the low absorbable piece 11 is 10 MPa or higher, and the compressive strength of the high absorbable piece 12 is 0.1 MPa or higher but 20 MPa or lower. The compressive strength of the low absorbable piece 11 is higher than that of the high absorbable piece 12, while the difference in the compressive strength between the low absorbable piece 11 and the high absorbable piece 12 is set to be 5 MPa or higher.

A strength of about 10 MPa is necessary for the low absorbable piece 11 and the high absorbable piece 12 to be pushed in to fill the defect 21 created by osteotomy or the like. In addition, a strength of 0.1 MPa is necessary for them to be formed in a block shape. Accordingly, it is possible, by forming the low absorbable piece 11 and the high absorbable piece 12 in the above-mentioned manner, for the low absorbable piece 11 to be pushed in to fill the defect 21, as well as being possible for the high absorbable piece 12 to be formed in a block shape.

The bioabsorption rate of the low absorbable piece 11 is from 1 to 5 years, provided that the size is 1 cm³; and the bioabsorption rate of the high absorbable piece 12 is from 0.25 to 2 years, provided that the size is 1 cm³.
By forming the low absorbable piece 11 and the high absorbable piece 12 in this manner, the low absorbable piece 11 having a porosity of, for example, 60% can be absorbed in 27 to 37 months, and the high absorbable piece 12 having a porosity of, for example, 75% can be absorbed in 2 years. Therefore, the defect 21 created by osteotomy or the like can be quickly repaired.

Hereunder is a description of the operation of the bone prosthetic material 1 having the above-mentioned structure.
Here, as shown in Fig. 2, the human bone is typically composed of an outer dense part and an inner coarse part. The outer dense part is referred to as the cortical bone 22 and the inner coarse part is referred to as the cancellous bone 23. The bone marrow 24 is housed within the cancellous bone 23. The cortical bone 22 is so rigid that it helps to support loads applied to the body. Although the cancellous bone 23 is not very rigid, it helps to keep the shape of the bone while saving the weight of the bone.

Blood circulation and cell migration are so active in the bone marrow 24 that the bone can be actively metabolized. Moreover, the center of a long bone such as femur and humerus is a medullary cavity where the cancellous bone 23 is not present but the bone marrow fluid is filled. Therefore, blood and cells are so abundant that the bone can be actively metabolized.
In this way, the human bone is characteristic in that the outer cortical bone 22 and the inner cancellous bone 23 have different rigidity and different speed of bone metabolism.

It is possible, by filling the bone prosthetic material 1 according to this embodiment in the defect 21 created by, for example, osteotomy so that the low absorbable piece 11 having a relatively low absorption rate is located on the cortical bone 22 side and the high absorbable piece 12 having a relatively high absorption rate is located on the cancellous bone 23 side, to fix the high absorbable piece 12 to the inside of the cancellous bone 23 right after the operation with the aid of the low absorbable piece 11 fixed to the cortical bone 22. Since the high absorbable piece 12 fixed to the inside of the cancellous bone 23 has a high bioabsorption rate, cells related to bone metabolism can act for angiogenesis, absorption of the material, bone formation, and such an event, by which the high absorbable piece 12 will be replaced with the autologous bone. Thereafter, the low absorbable piece 11 fixed to the inside of the cortical bone 22 will also be replaced with the autologous bone in the same manner. By so doing, the defect 21 created by osteotomy can be quickly repaired.

In this case, because the low absorbable piece 11 and the high absorbable piece 12 are connected by the joint 14 that limits displacement in directions along the connecting surface 13, it is possible, by inserting them into the defect 21 in a direction orthogonal to the connecting surface 13 as the insertion direction, to prevent positional misalignment of the low absorbable piece 11 and the high absorbable piece 12 in directions along the connecting surface 13.
Moreover, because the low absorbable piece 11 and the high absorbable piece 12 are connected by the joint 14, each of the low absorbable piece 11 and the high absorbable piece 12 can be sufficiently hardened by means of burning or such a treatment prior to the connection, and the low absorbable piece 11 and the high absorbable piece 12 can be prevented from being disconnected due to the difference in the coefficient of thermal expansion after the connection.
In addition, because the low absorbable piece 11 and the high absorbable piece 12 are integrated, the work to insert the low absorbable piece 11 and the high absorbable piece 12 can be done at once. By so doing, the operation can be facilitated and also the time required therefor can be shortened, by which the burden on the patient can be alleviated.

Note that, as shown in Fig. 3, the joint 14 may also comprise a recess 15 provided in the high absorbable piece 12 and a projection 16 provided on the low absorbable piece 11 to be engaged with the recess 15. Moreover, as shown in Fig. 4, the joint 14 may also comprise a projection 16 and a recess 15 on the low absorbable piece 11 and in the high absorbable piece 12 at a position decentered from the center of the connecting surface 13.

Furthermore, as shown in Fig. 5, the joint 14 may also comprise a female screw formed on the recess 15 and a male screw formed on the projection 16.
By so doing, it is possible to make the low absorbable piece 11 and the high absorbable piece 12 detachable/attachable in a simple manner, and it is also possible to prevent displacement of the low absorbable piece 11 and the high absorbable piece 12 not only in directions along the connecting surface 13 but also in directions orthogonal to the connecting surface 13, that is to say, the direction to insert the bone prosthetic material 1 into the defect 21. Thereby, the workability for insertion into the defect 21 can be improved.

Moreover, as shown in Fig. 6, the joint 14 may also comprises a plurality of recesses 15a and 15b provided in the low absorbable piece 11 and a plurality of projections 16a and 16b provided on the high absorbable piece 12 to be engaged with the recesses 15a and 15b. By having such a joint 14, the contact areas between the recesses 15a and 15b and the projections 16a and 16b can be increased, by which the frictional force can be enhanced. By so doing, misalignment of the low absorbable piece 11 and the high absorbable piece 12 in the insertion direction (direction orthogonal to the connecting surface 13) can be made unlikely to occur when inserting the bone prosthetic material 1 into the defect 21.

As shown in Fig. 7, the joint 14 may also be such that the curvature radius of the basal end of the projection 16 is larger than the curvature radius of the open end of the recess 15. As the difference of this curvature radius is greater, the frictional force between the projection 16 and the recess 15 can be increased. By so doing, misalignment of the low absorbable piece 11 and the high absorbable piece 12 in the insertion direction (direction orthogonal to the connecting surface 13) can be made unlikely to occur when inserting the bone prosthetic material 1 into the defect 21.

As shown in Fig. 8, the joint 14 may also be such that the cross sectional area of the recess 15 is decreased towards the bottom face and the cross sectional area of the projection 16 is decreased towards the top end. By so doing, the low absorbable piece 11 and the high absorbable piece 12 can be detached/attached in a simple manner.

As shown in Fig. 9, the joint 14 may also be such that the cross sectional area of the recess 15 is increased towards the bottom face and the cross sectional area of the projection 16 is increased towards the top end. By so doing, it is possible to prevent changes of their relative positions not only in directions along the connecting surface 13 but also in directions orthogonal to the connecting surface 13. Therefore, the workability for insertion into the defect 21 can be improved.

As shown in Fig. 10, the joint 14 may also be formed in a shape in which the recess 15 and the projection 16 do not pierce throughout the width direction (the direction orthogonal to the plane of Fig. 10) of the low absorbable piece 11 and the high absorbable piece 12. In particular, it is possible, by forming the recess 15 to pierce through one of the lateral faces only, to avoid a mistake regarding the orientation to connect the low absorbable piece 11 and the high absorbable piece 12.

As shown in Fig. 11, the joint 14 may also be formed such that the projection 16 and the recess 15 are oriented in a direction orthogonal to the bottom faces of the low absorbable piece 11 and the high absorbable piece 12.
Further, as shown in Fig. 12, if three or more of prosthetic material pieces are connected, joints 14a and 14b may also be formed in the respective connecting surfaces.

As shown in Fig. 13, the joint 14 may also comprise recesses 15a and 15b provided in both the low absorbable piece 11 and the high absorbable piece 12, and a joining member 18 to be engaged with these recesses 15 to thereby connect the low absorbable piece 11 and the high absorbable piece 12.
By having such a joint, the material of the joining member 18 can be selected irrespective of the materials of the low absorbable piece 11 and the high absorbable piece 12 so that the joining member 18 can have a desired strength to thereby enhance the strength to bond the low absorbable piece 11 and the high absorbable piece 12.

Furthermore, as shown in Fig. 14, it is also possible such that recesses are formed respectively in the connecting surfaces of the low absorbable piece 11 and the high absorbable piece 12, and a joining member 19 having projections that can engage with these recesses is provided between the low absorbable piece 11 and the high absorbable piece 12. In this case, the joining member 19 plays a role as a bonding film that connects the low absorbable piece 11 and the high absorbable piece 12 while preventing a direct contact therebetween. Note that, though it is not shown, a similar effect can also be given by forming projections respectively on the connecting surfaces of the low absorbable piece 11 and the high absorbable piece 12, and providing such a joining member 19 having recesses that can engage with these projections between the low absorbable piece 11 and the high absorbable piece 12.

The connecting surfaces 13 between the low absorbable piece 11 and the high absorbable piece 12 can also be coated with a biocompatible bonding material.
This bonding material can be exemplified by, for example, a cement, a ceramic slurry, a collagen, a gelatin, fibrin, chitin, chitosan, hyaluronic acid, chondroitin sulfate, alginic acid, polylactic acid, polyglycolic acid, polyacrylic acid, polycaprolactone, a cellulose modifier, dextrose, dextran/poly(vinyl alcohol), polyethylene, polypropylene, ficoll, blood, cells, extracellular matrix, a growth factor, bone, bone marrow, a ceramic (alumina or zirconia), a metal (titanium, a stainless steel, or an alloy thereof), a carbon, a modified substance thereof, or a composite thereof.

Moreover, in order to enhance the strength to bond the low absorbable piece 11 and the high absorbable piece 12, a heat treatment can also be applied under a condition in which each of the burned or hardened prosthetic material pieces would not shrink. In this case, the temperature of the heat treatment has to be set below the temperature for burning, for example, the low absorbable piece 11 and the high absorbable piece 12.
In order to control the absorption rates of the low absorbable piece 11 and the high absorbable piece 12, a biocompatible material such as mentioned above may also be mixed or coated.

In this embodiment, the description has been made by referring to a case where the bone prosthetic material 1 is filled in the defect 21 created by osteotomy. However, the present invention is also applicable to other defects created by fracture of the distal radius or tumor ablation.

### {First Example}

Hereunder, specific structures of the above-mentioned bone prosthetic material 1 will be explained.
As shown in Fig. 15 and Fig. 16, the bone prosthetic material 1 according to the first example has a shape like a triangular prism which has faces of a height of 7 mm, a depth of 30 mm, and an angle of 6°. The bone prosthetic material 1 comprises tricalcium phosphate as a bioabsorbable material.

The low absorbable piece 11 having a low absorption/replacement rate is a structural body having a porosity of 60% and a compressive strength of 20 MPa, in which micropores of 1 to 1000 nm account for 83% (bulk ratio) and micropores of 1 to 200 µm account for 17% (bulk ratio). The high absorbable piece 12 having a high absorption/replacement rate is a structural body having a porosity of 75% and a compressive strength of 3 MPa, in which micropores of 1 to 1000 nm account for 57% (bulk ratio) and micropores of 1 to 200 µm account for 43% (bulk ratio).

The low absorbable piece 11 is shaped like a quadrangular prism which has faces of a height of 7 mm and a depth of 10 mm, and has a recess 15 of a height of 2.6 mm and a depth of 1.5 mm that pierces throughout the width direction. The high absorbable piece 12 is shaped like a triangular prism which has faces of a height of about 4.7 mm and a depth of 20 mm, and has a projection 16 of a height of 2.6 mm and a depth of 1.5 mm that engages with the recess 15.

### {Second Example}

As shown in Fig. 17, the bone prosthetic material 2 according to the second example has a shape like a quadrangular prism having a height of 10 mm and a depth of 20 mm. The bone prosthetic material 1 comprises tricalcium phosphate as a bioabsorbable material, similarly to the first example.

The low absorbable piece 11 having a low absorption/replacement rate is a structural body having a porosity of 60% and a compressive strength of 20 MPa, in which micropores of 1 to 1000 nm account for 83% (bulk ratio) and micropores of 1 to 200 µm account for 17% (bulk ratio). The high absorbable piece 12 having a high absorption/replacement rate is a structural body having a porosity of 75% and a compressive strength of 3 MPa, in which micropores of 1 to 1000 nm account for 57% (bulk ratio) and micropores of 1 to 200 µm account for 43% (bulk ratio).

The low absorbable piece 11 is shaped like a quadrangular prism which has faces of a height of 10 mm and a depth of 10 mm, and has a recess 15 of a height of 3.0 mm and a depth of 3.0 mm. The high absorbable piece 12 is shaped like a quadrangular prism which has faces of a height of 10 mm and a depth of 10 mm, and has a projection 16 of a height of 3.0 mm and a depth of 3.0 mm that engages with the recess 15.

### {Third Example}

As shown in Fig. 18, the bone prosthetic material 3 according to the third example has a shape like a circular column having a diameter of 10 mm and a depth (height) of 20 mm. The bone prosthetic material 1 comprises tricalcium phosphate as a bioabsorbable material, similarly to the first example.

The low absorbable piece 11 is shaped like a circular column having a height of 10 mm and a depth (height) of 10 mm, and has a recess 15 having a diameter ϕ of 3.0 mm and a depth of 3.0 mm. The high absorbable piece 12 is shaped like a circular column having a height of 10 mm and a depth (height) of 10 mm, and has a projection 16 having a diameter ϕ of 3.0 mm and a depth of 3.0 mm that engages with the recess 15.

Note that, although it is not necessary to provide the recess 15 and the projection 16 on the central axis of the low absorbable piece 11 and the high absorbable piece 12, it is necessary in this case to take a countermeasure to prevent rotation so that the low absorbable piece 11 and the high absorbable piece 12 would not relatively rotate about the central axis, wherein the countermeasure can be exemplified by providing pluralities of numbers of recesses 15 and projections 16, or the like. Moreover, as for the countermeasure to prevent rotation, it is also possible to form the recess 15 and the projection 16 in shapes like quadrangular prisms or such polygonal shapes, rather than shapes like circular columns.

### {Fourth Example}

The bone prosthetic material 4 according to the fourth example has a triangular cross sectional shape, similarly to the first example. As shown in Fig. 19, the bone prosthetic material 4 is shaped like a crescent when viewed from the top. The outer edge of the crescent is disposed a low absorbable piece 11, and the inner edge thereof is disposed a high absorbable piece 12.

In osteotomy, the cutting has to be made broad sometimes depending on the case of the patient. In such a case, it is necessary to insert a plurality of numbers of the bone prosthetic materials 1 of the first example, for example. In this case, according to the bone prosthetic material 4 of this example, it suffices only to insert one bone prosthetic material 4 into the defect 21. Therefore, the operation can be facilitated and also the time required therefor can be shortened, by which the burden on the patient can be alleviated.

### {Production Method}

Next is a description of the method for producing the above-mentioned bone prosthetic material 1 to 4. Here, as an example, a method for producing the bone prosthetic material 1 according to the first example will be described.
Fig. 20 shows the method for producing the bone prosthetic material 1 by means of a slurry foaming method.
As shown in Fig. 20, firstly, a calcium phosphate powder and a liquid agent (such as a dispersing agent or a foaming agent) are mixed (Step S11). In this case, the viscosity of the mixed liquid (slurry) varies depending on the powder-to-liquid ratio or the mixing time. The lower this viscosity is, the higher the porosity will be.

Next, air bubbles are made by foaming (Step S12). In this case, the longer the foaming time is, the higher the porosity will be. In other words, the porosity of the high absorbable piece 12 can be set higher than that of the low absorbable piece 11 by ensuring the foaming time for the high absorbable piece 12 to be longer than the foaming time for the low absorbable piece 11. By so doing, the bioabsorption rate of the high absorbable piece 12 can be set higher than the bioabsorption rate of the low absorbable piece 11.

Next, organic compounds are removed by heating and particulates are made bound (Step S13). By so doing, each of the low absorbable piece 11 and the high absorbable piece 12 can be sufficiently hardened (burned). This makes it possible to prevent the low absorbable piece 11 and the high absorbable piece 12 from being disconnected due to the difference in the coefficient of thermal expansion after the low absorbable piece 11 and the high absorbable piece 12 have been connected. Note that the larger the heat quantity (temperature and time) is applied, the lower the porosity will be.

Next, a recess 15 is formed in the low absorbable piece 11 and a projection 16 to be engaged with the recess 15 is formed on the high absorbable piece 12, by means of machining (Step S14).
Then, the projection 16 is engaged with the recess 15 to thereby connect the low absorbable piece 11 and the high absorbable piece 12 (Step S15). By so doing, the low absorbable piece 11 and the high absorbable piece 12 can be integrally connected so that displacement in directions along the connecting surface 13 can be prevented.

In the above-mentioned production method, the procedure may also be such that, as shown in Fig. 21, after the completion of the foaming process of Step S12, the recess 15 is formed in the low absorbable piece 11 and the projection 16 to be engaged with the recess 15 is formed on the high absorbable piece 12, by means of molding (Step S17). In this case, it is necessary to determine the size of the mold with considering the shrinkage that occurs during the heating process of Step S13.

Further, as shown in Fig. 22, it is also possible to skip the foaming process of Step S12 by adding a cavitating agent in the mixing process (Step S11) of Fig. 20 (method of adding a cavitating agent). Similarly, as shown in Fig. 23, it is also possible to skip the foaming process of Step S12 by adding a cavitating agent in the mixing process (Step S11) of Fig. 21.

Moreover, as shown in Fig. 24, it is also possible to conduct a second mixing step (Step S18) in which a template, for example, a porous sponge and a slurry are mixed (template method), after the completion of the mixing process (Step S11) of Fig. 20. The porosities of the low absorbable piece 11 and the high absorbable piece 12 vary depending on the shape of this template. Note that it is either possible to coat the slurry so that the framework of the template can remain, or to impregnate the inside of the template with the slurry so that the framework of the template can serve as the cavity.

Furthermore, as shown in Fig. 25, it is also possible to mix the calcium phosphate powder and a hardening agent (for example, alginate or gypsum) in the mixing process (Step S11) of Fig. 20 (method of adding a hardening agent). Then, the mixed liquid is poured into a mold and time is allowed until hardening is completed in a molding and hardening step (Step S19).

As shown in Fig. 26, the hardening can also be achieved by pouring the mixed liquid into the mold and allowing time in the molding and hardening step (Step S19) (dissolution and reprecipitation method), instead of adding a hardening agent in the mixing process (Step S11) of Fig. 25. In this method, the mixed liquid is hardened through dissolution and reprecipitation of calcium phosphate.

As described above, embodiments of the present invention have been explained in detail with reference to the drawings. However, the specific structure is not to be limited to these embodiments, and includes modifications of the design without departing from the sprit or scope of the present invention.
For example, the description has been made by setting a situation in which the bone prosthetic materials 1 to 4 respectively comprise two prosthetic material pieces, namely, the low absorbable piece 11 and the high absorbable piece 12. However, the bone prosthetic materials 1 to 4 may also comprise three or more prosthetic material pieces.
Moreover, the description has been made by showing that the bone prosthetic materials 1 to 4 are respectively shaped like a triangular prism, a quadrangular prism, a circular column, and a crescent (when view from the top). However, the bone prosthetic materials 1 to 4 are not to be limited to these shapes, and various types of shapes can be selected corresponding to the shape of the defect 21.

### {Reference Signs List}

- 1, 2, 3, and 4:: Bone prosthetic material
- 11:: Low absorbable piece
- 12:: High absorbable piece
- 13:: Connecting surface
- 14, 14a, and 14b:: Joint
- 15, 15a, and 15b:: Recess
- 16, 16a, and 16b:: Projection
- 18:: Joining member
- 21:: Defect
- 22:: Cortical bone
- 23:: Cancellous bone
- 24:: Bone marrow

## Claims

1. A bone prosthetic material comprising:
a plurality of prosthetic material pieces which include bioabsorbable materials having different absorption rates, and which are adjacent and connected to each other; and
a joint which is provided in a connecting surface between the prosthetic material pieces, and which is mutually combined to connect the prosthetic material pieces while limiting displacement in a direction along the connecting surface.

2. A bone prosthetic material according to claim 1, wherein the joint comprises:
a recess which is provided in one of the adjacent prosthetic material pieces; and
a projection which is provided on another one of the adjacent prosthetic material pieces to be engaged with the recess.

3. A bone prosthetic material according to claim 2, wherein a female screw is formed on the recess and a male screw is formed on the projection.

4. A bone prosthetic material according to claim 1, wherein the joint comprises:
recesses provided in each of the adjacent prosthetic material pieces; and
a joining member to be engaged with the recesses to thereby connect the adjacent prosthetic material pieces.

5. A bone prosthetic material according to any one of claim 1 to claim 4, which is formed in a wedge shape so that the cross sectional area gradually decreases from one end of a prosthetic material piece having a relatively low absorption rate towards one end of a prosthetic material piece having a relatively high absorption rate, among the adjacent prosthetic material pieces.

6. A bone prosthetic material according to any one of claim 1 to claim 5, wherein the prosthetic material pieces are formed from a calcium phosphate based compound, a calcium sulfate based compound, a calcium carbonate based compound, or a compound in which a part of elements thereof is substituted with other elements, or a ceramic which includes a composite of these compounds as a main component.

7. A bone prosthetic material according to any one of claim 1 to claim 6, wherein:
a low absorbable piece serving as a prosthetic material piece having a relatively low absorption rate, out of the adjacent prosthetic material pieces, is a porous body whose porosity is 70% or lower;
a high absorbable piece serving as a prosthetic material piece having a relatively high absorption rate, out of the adjacent prosthetic material pieces, is a porous body whose porosity is 50% or higher but 90% or lower; and
the porosity of the high absorbable piece is higher than the porosity of the low absorbable piece, while the difference in the porosity between them is 10% or larger.

8. A bone prosthetic material according to claim 7, wherein:
the low absorbable piece includes pores having a radius of 1 nm or larger but smaller than 1000 nm, which account for 80% or more in the volume ratio, and pores having a radius of 1 µm or larger but 100 µm or smaller, which account for less than 20% in the volume ratio; and
the high absorbable piece includes pores having a radius of 1 nm or larger but smaller than 1000 nm, which account for 50% or more in the volume ratio, and pores having a radius of 1 µm or larger but 100 µm or smaller, which account for less than 50% in the volume ratio.

9. A bone prosthetic material according to any one of claim 1 to claim 8, wherein:
the compressive strength of a low absorbable piece serving as a prosthetic material piece having a relatively low absorption rate, out of the adjacent prosthetic material pieces, is 10 MPa or higher;
the compressive strength of a high absorbable piece serving as a prosthetic material piece having a relatively high absorption rate, out of the adjacent prosthetic material pieces, is 0.1 MPa or higher but 20 MPa or lower; and
the compressive strength of the low absorbable piece is higher than that of the high absorbable piece, while the difference in the compressive strength between the low absorbable piece and the high absorbable piece is 5 MPa or higher.

10. A bone prosthetic material according to any one of claim 1 to claim 9, wherein:
the bioabsorption rate of a low absorbable piece serving as a prosthetic material piece having a relatively low absorption rate, out of the adjacent prosthetic material pieces, is from 1 to 5 years, provided that the size is 1 cm³; and
the bioabsorption rate of a high absorbable piece serving as a prosthetic material piece having a relatively high absorption rate, out of the adjacent prosthetic material pieces, is from 0.25 to 2 years, provided that the size is 1 cm³.

11. A bone prosthetic material according to claim 5, wherein:
the longitudinal length from one end to the other end throughout the plurality of the prosthetic material pieces, after connected, is from 3 mm to 40 mm;
the length in a direction orthogonal to the longitudinal direction of the plurality of the prosthetic material pieces, after connected, is from 3 mm to 30 mm; and
the pieces are to be filled in a defect created in a cortical bone and a cancellous bone.
